# EUROPÄISCHE PATENTANMELDUNG

(11) **EP 3 323 340 A1**
(43) Veröffentlichungstag der Anmeldung: **23.05.2018**
(21) Anmeldenummer: 17201874.9
(22) Anmeldetag: 15.11.2017
(51) Int. Cl.: A61B 1/07, A61B 1/00, A61B 5/00

(54) **ENDOSKOPISCHE SONDE, SYSTEM UND VERFAHREN FÜR OPTISCHE KOHÄRENZTOMOGRAPHIE UND KONFOKALE ENDOSKOPIE**

(30) Priorität: 16.11.2016 DE 102016121984
(71) Anmelder: Karl Storz SE & Co. KG, 78532 Tuttlingen (DE)
(72) Erfinder: Bechtel, Christin, 78532 Tuttlingen (DE)

(57) **Zusammenfassung**

Eine erfindungsgemäße endoskopische Sonde für die optische Kohärenztomographie und die konfokale Endoskopie umfasst einen Lichtleiter zum Weiterleiten einer ersten Beleuchtungsstrahlung für die Kohärenztomographie, die einen ersten Wellenlängenbereich umfasst, und einer zweiten Beleuchtungsstrahlung für die konfokale Endoskopie, die einen zweiten Wellenlängenbereich umfasst, von einer proximalen zu einer distalen Endfläche (25) des Lichtleiters, sowie ein Objektiv (17) zum Weiterleiten der ersten und zweiten Beleuchtungsstrahlung von der distalen Endfläche (25) des Lichtleiters zu einem zu beobachtenden Objektbereich, und zum Weiterleiten einer von dem Objektbereich ausgehenden Beobachtungsstrahlung zur distalen Endfläche (25) des Lichtleiters. Das Objektiv (17) umfasst eine wellenlängenabhängige Blende (27), die im ersten Wellenlängenbereich eine kleinere Blendenöffnung hat als im zweiten Wellenlängenbereich. Der Lichtleiter ist ferner zur Weiterleitung der eingekoppelten Beobachtungsstrahlung zur proximalen Endfläche (14) und als einzelne Lichtleitfaser ausgebildet, und die distale Endfläche (25) der Lichtleitfaser ist quer zur optischen Achse (29) des Objektivs (17) bewegbar. Die Erfindung betrifft auch ein System und ein Verfahren zur kombinierten optischen Kohärenztomographie und konfokalen Endoskopie.

## Beschreibung

Die vorliegende Erfindung betrifft eine endoskopische Sonde für optische Kohärenztomographie und konfokale Endoskopie nach dem Oberbegriff von Anspruch 1, sowie ein System und ein Verfahren zur optischen Kohärenztomographie und konfokalen Endoskopie.

Aus US 2011/0261367 A1 ist ein integriertes konfokales und optisches Kohärenztomographie-Mikroskop bekannt, wobei für die beiden Verfahren unterschiedliche Wellenlängenbereiche genutzt werden. Ein optisches Faserbündel wird genutzt, um Beleuchtungslicht zu einem Objekt zu übertragen und rückkommendes Licht von dem Objekt für die konfokale Mikroskopie und die optische Kohärenztomographie bereit zu stellen. Um sowohl einen Beleuchtungsstrahl mit hoher numerischer Apertur für die konfokale Mikroendoskopie als auch einen Beleuchtungsstrahl mit niedriger numerischer Apertur für die optische Kohärenztomographie zu nutzen, wird in einem Miniaturobjektiv mit hoher numerischer Apertur ein ringförmiger Filter verwendet, wobei der Filter nur die infraroten Wellenlängen der optischen Kohärenztomographie abblockt, aber die sichtbaren Wellenlängen des konfokalen Beleuchtungsstrahls und zurückkommender Fluoreszenzstrahlung des konfokalen Mikroendoskops durchlässt. Zum Scannen des Beobachtungsobjekts ist am proximalen Ende des Faserbündels ein beweglicher Scanspiegel vorgesehen, der eine durch eine Schlitzblende erzeugte Beleuchtungslinie über die proximale Endfläche des Faserbündels bewegt.

In dem Artikel "Dual-modality fiber-based OCT-TPL imaging system for simultaneous microstructural and molecular analysis of atherosclerotic plaques" von Wang et al., Biomed. Opt. Express 6 (5), 1665-1678 (2015) ist ein faserbasiertes optisches abbildendes System offenbart, das optische Kohärenztomographie und Zwei-Photonen-Lumineszenz-Bildgebung kombiniert. Dabei wird eine Photonikkristall-Faser zur Übertragung des Beleuchtungslichts zur Probe und zur Rückübertragung des von der Probe emittierten Lichts genutzt.

Bei den bekannten Lösungen ist keine gleichzeitige Aufnahme hochaufgelöster Tiefendaten und endomikroskopischer Bilddaten möglich. Eine solche gleichzeitige Aufnahme von Tiefen- und endomikroskopischen Bilddaten wäre jedoch beispielsweise zur Feststellung zellulärer Veränderungen innerhalb eines Gewebebereichs wünschenswert.

Es ist Aufgabe der vorliegenden Erfindung, eine endoskopische Sonde für optische Kohärenztomographie und konfokale Endoskopie sowie ein entsprechendes System und ein Verfahren anzugeben, wobei insbesondere die genannten Nachteile vermieden werden.

Diese Aufgabe wird durch eine endoskopische Sonde gemäß Anspruch 1 sowie durch ein System gemäß Anspruch 9 und ein Verfahren gemäß Anspruch 13 gelöst.

Vorteilhafte Weiterbildungen der Erfindung ergeben sich aus den Unteransprüchen.

Eine erfindungsgemäße endoskopische Sonde ist sowohl für die Durchführung der optischen Kohärenztomographie als auch der konfokalen Endoskopie geeignet. Die Sonde ist als optische Sonde für eine Untersuchung eines Bereichs eines Objekts mittels einer Kombination von optischer Kohärenztomographie und konfokaler Endoskopie ausgebildet. Der Objektbereich kann ein Bereich eines menschlichen oder tierischen Gewebes sein, insbesondere ein Gewebereich in einem körperinneren Hohlraum eines menschlichen oder tierischen Körpers. Im Folgenden wird die Erfindung für die Beobachtung eines Gewebebereichs eines menschlichen oder tierischen Gewebes mittels kombinierter optischer Kohärenztomographie und konfokaler Endoskopie beschrieben. Die Erfindung kann jedoch auch für nicht-medizinische Anwendungen vorteilhaft sein, beispielsweise für die Untersuchung eines Objektbereichs eines technischen Objekts, insbesondere eines teilweise transparenten Objektbereichs.

Bei der optischen Kohärenztomographie (Optical Coherence Tomography, OCT) wird eine Beleuchtungsstrahlung geringer Kohärenzlänge in einem Messarm auf ein Untersuchungsobjekt eingestrahlt und die vom Untersuchungsobjekt zurückkommende Beobachtungsstrahlung mit einem in einen Referenzarm eingekoppelten Anteil der Beleuchtungsstrahlung zur Interferenz gebracht. Aufgrund der kurzen Kohärenzlänge kann aus den auftretenden Interferenzen auf die optische Weglänge des Messarms und damit auf optische Strukturen innerhalb des Untersuchungsobjekts in Abhängigkeit von der Tiefe geschlossen werden. Mittels OCT kann ein eindimensionales, in Strahlrichtung gerichtetes Tiefenprofil gewonnen werden, sowie durch ein- oder zweidimensionales Abtasten (Scannen) ein axiales Schnittbild bzw. ein 3D-Tiefenprofil des Untersuchungsobjekts erzeugt werden. Bei Untersuchungen menschlichen oder tierischen Gewebes hat die optische Kohärenztomographie den Vorteil, dass Gewebestrukturen typischerweise bis zu einer Tiefe von einigen Millimetern mit einer lateralen Auflösung im Bereich von etwa 10 bis 30 µm erfasst werden können. Die optische Kohärenztomographie kann auch endoskopisch angewendet werden. Vorrichtungen und Verfahren für die optische Kohärenztomographie sind an sich bekannt.

Bei der konfokalen Mikroskopie wird eine Beleuchtungsstrahlung auf einen Punkt in einer Fokusebene, die an oder nahe an einer Oberfläche eines zu untersuchenden Objekts liegt, fokussiert. Die von diesem beleuchteten Objektpunkt ausgehende Beobachtungsstrahlung wird von der gleichen Optik aufgenommen und beispielsweise auf eine Lochblende abgebildet, hinter der ein Detektor angeordnet ist. Strahlung, die nicht aus der Fokusebene kommt, wird dadurch weitgehend ausgeblendet. Auf diese Weise kann eine erhöhte Auflösung erzielt werden, insbesondere dann, wenn die Beleuchtung und die Beobachtung mit einer hohen numerischen Apertur erfolgen. Hierbei erfolgt ein punktweises Abtasten des Untersuchungsobjekts durch ein- oder zweidimensionales Scannen, um ein ein- oder zweidimensionales Bild zu gewinnen. Die konfokale Mikroskopie verbindet eine hohe Auflösung in lateraler Richtung quer zu einer optischen Achse der Beleuchtung mit einer vergleichbar hohen Auflösung in axialer Richtung. Menschliches oder tierisches Gewebe ist mit konfokaler Mikroskopie typischerweise bis zu einer Tiefe von wenigen 100 µm darstellbar. Die konfokale Mikroskopie ist auch endoskopisch durchführbar und wird dann als konfokale Endomikroskopie oder als konfokale Endoskopie bezeichnet. Untersuchungsverfahren, die ebenfalls konfokal arbeiten und auf die Detektion von Fluoreszenz gerichtet sind, einschließlich der Multiphotonenmikroskopie oder der Detektion nichtlinearer Effekte, werden im Folgenden ebenfalls als konfokale Endoskopie zusammengefasst. Vorrichtungen und Verfahren für die konfokale Endoskopie sind an sich bekannt.

Eine erfindungsgemäße endoskopische Sonde für die optische Kohärenztomographie und die konfokale Endoskopie umfasst einen langerstreckten Lichtleiter, der zum Weiterleiten einer ersten Beleuchtungsstrahlung, die einen ersten Wellenlängenbereich umfasst und als Beleuchtungsstrahlung bei der Kohärenztomographie geeignet ist, und einer zweiten Beleuchtungsstrahlung, die einen zweiten Wellenlängenbereich umfasst und als Beleuchtungsstrahlung für die konfokale Endoskopie geeignet ist, von einer proximalen (benutzernahen) Endfläche des Lichtleiters zu einer distalen (benutzerfernen) Endfläche des Lichtleiters ausgebildet ist. Da bei der Kohärenztomographie eine Kohärenz der Beleuchtungsstrahlung notwendig ist, insbesondere ein für die Detektion von Interferenzen ausreichender Kohärenzgrad und eine für die Erzielung der gewünschten axialen Auflösung geeignete Kohärenzlänge, ist der Lichtleiter zum Weiterleiten unter zumindest teilweiser Erhaltung der Kohärenz der in die proximale Endfläche einkoppelbaren ersten Beleuchtungsstrahlung ausgebildet. Weiter kann der Lichtleiter zum Weiterleiten der in die proximale Endfläche einkoppelbaren zweiten Beleuchtungsstrahlung mit einer hohen numerischen Apertur geeignet sein. Aus der distalen Endfläche des Lichtleiters können die erste und die zweite Beleuchtungsstrahlung beispielsweise in axialer Richtung austreten. Der erste und der zweite Wellenlängenbereich sind unterschiedliche Wellenlängenbereiche, vorzugsweise nicht-überlappende Bereiche im sichtbaren und/oder in angrenzenden Bereichen des elektromagnetischen Spektrums. Beispielsweise kann der erste Wellenlängenbereich im infraroten oder im längerwelligen Teil des sichtbaren Spektralbereichs liegen und der zweite Wellenlängenbereich den grünen und/oder blauen Wellenlängenbereich umfassen. Im Rahmen der vorliegenden Erfindung wird unter "Licht" elektromagnetische Strahlung im sichtbaren Spektralbereich sowie in angrenzenden Spektralbereichen, insbesondere im infraroten (IR) und/oder ultravioletten (UV) Spektralbereich, verstanden. Im gleichen Sinne wird der Ausdruck "Strahlung" verwendet.

Weiter umfasst die erfindungsgemäße Sonde ein Objektiv zum Weiterleiten der ersten und der zweiten Beleuchtungsstrahlung von der distalen Endfläche des Lichtleiters zu einem zu beobachtenden Gewebebereich. Insbesondere werden die erste und die zweite Beleuchtungsstrahlung, die aus der distalen Endfläche des Lichtleiters austreten, in eine Fokusebene fokussiert, die an oder in dem zu untersuchenden Gewebebereich liegt. Das Objektiv ist ferner zum Weiterleiten einer von dem Gewebebereich ausgehenden Beobachtungsstrahlung zur distalen Endfläche des Lichtleiters ausgebildet. Insbesondere ist das Objektiv zum Aufnehmen der vom Gewebebereich ausgehenden Beobachtungsstrahlung und zum Fokussieren der aufgenommenen Beobachtungsstrahlung auf die distale Endfläche des Lichtleiters ausgebildet und angeordnet. Hierdurch kann eine vom Gewebebereich aufgrund der eingestrahlten Beleuchtungsstrahlung emittierte Beobachtungsstrahlung in den Lichtleiter eingekoppelt werden.

Das Objektiv umfasst eine wellenlängenabhängig wirkende Blende, die im ersten Wellenlängenbereich eine kleinere Blendenöffnung hat als im zweiten Wellenlängenbereich. Hierdurch kann es erreicht werden, dass die erste Beleuchtungsstrahlung mit einer kleineren numerischen Apertur auf den zu untersuchenden Gewebebereich eingestrahlt wird als die zweite Beleuchtungsstrahlung. Da für die konfokale Endoskopie eine große numerische Apertur günstig ist, um eine hohe Auflösung zu erzielen, und andererseits für die optische Kohärenztomographie eine kleine numerische Apertur vorteilhaft ist, um eine hohe Eindringtiefe zu erzielen, können auf diese Weise sowohl die konfokale Endoskopie als auch die optische Kohärenztomographie mit einer jeweils angepassten numerischen Apertur der Beleuchtung durchgeführt werden. Insbesondere kann es erreichbar sein, dass sowohl die Einstrahlung der jeweiligen Beleuchtungsstrahlung auf den zu untersuchenden Gewebebereich als auch die Aufnahme und Fokussierung der Beobachtungsstrahlung auf die distale Endfläche des Lichtleiters mit einer jeweils optimalen numerischen Apertur erfolgen. Die hohe numerische Apertur für die zweite Beleuchtungsstrahlung zur Durchführung der konfokalen Mikroskopie ist beispielsweise größer 0,4 oder größer 0,8 und insbesondere größer oder gleich 1,0. Die durch die Blende erreichte geringe numerische Apertur für die erste Beleuchtungsstrahlung zur Durchführung der OCT ist beispielsweise kleiner als 0,3 und insbesondere kleiner 0,1 . Die wellenlängenabhängige Blende kann in vorteilhafter Weise in oder in der Nähe einer Hauptebene des Objektivs angeordnet sein. Die Blende kann insbesondere auf einer optischen Fläche einer Linse des Objektivs angeordnet sein.

Der Lichtleiter ist weiterhin zur Weiterleitung der eingekoppelten Beobachtungsstrahlung zur proximalen Endfläche des Lichtleiters ausgebildet, von wo diese beispielsweise zu einer Detektoreinrichtung weitergeleitet und zur Erzeugung von Bilddaten genutzt werden kann. Hierfür kann der Lichtleiter auch bei der Weiterleitung der Beobachtungsstrahlung zum proximalen Ende zur zumindest teilweisen Erhaltung der Kohärenz sowie zur Weiterleitung einer mit hoher numerischer Apertur eingekoppelten Beobachtungsstrahlung ausgebildet sein.

Erfindungsgemäß ist der Lichtleiter als eine einzelne Lichtleitfaser ausgebildet. Diese Lichtleitfaser dient somit zur Weiterleitung der Beleuchtungsstrahlung in distaler Richtung und zur Weiterleitung der Beobachtungsstrahlung in proximaler Richtung, und zwar sowohl für die optische Kohärenztomographie als auch für die konfokale Endoskopie. Ferner ist es erfindungsgemäß vorgesehen, dass die distale Endfläche des Lichtleiters, d.h. die distale Endfläche der einzelnen Lichtleitfaser, quer zur optischen Achse des Objektivs bewegbar ist. Insbesondere ist ein distaler Endabschnitt der Lichtleitfaser in mindestens einer Querrichtung der Lichtleitfaser bewegbar. Hierfür kann in an sich bekannter Weise ein motorischer Antrieb, beispielsweise ein piezoelektrischer Antrieb und/oder ein resonanter Antrieb, vorgesehen sein.

Dadurch, dass der Lichtleiter durch nur eine einzige Lichtleitfaser gebildet wird, ist eine besonders hohe Auflösung erreichbar, insbesondere ist die Aufnahme endomikroskopischer Bilddaten mit einer besonders hohen lateralen und ggf. auch axialen Auflösung erreichbar. Die Bilddaten stellen insbesondere die Reflexions-, Streuungs- und/oder Fluoreszenzeigenschaften des Gewebebereichs bzw. der beleuchteten Objektpunkte dar. Dabei kann die Auflösung vom Durchmesser der Lichtleitfaser abhängen und ist in der Regel umso höher, je geringer der Durchmesser ist. Ferner kann dadurch, dass nur eine einzige Lichtleitfaser zur Weiterleitung der Beleuchtungs- und der Beobachtungsstrahlung vorgesehen ist, die Sonde mit einem besonders geringen Durchmesser ausgestaltet werden. Die Sonde kann daher für einen portablen Einsatz sowie für eine Integration in ein Endoskop geeignet sein.

Dadurch, dass die distale Endfläche der einzelnen Lichtleitfaser quer zur optischen Achse des Objektivs bewegbar ist, insbesondere motorisch bewegbar ist, wird ein Scannen des beobachteten Gewebebereichs ermöglicht. Hierdurch können sequenziell unterschiedliche Objektpunkte des Gewebebereichs beleuchtet und beobachtet werden. Insbesondere wird durch eine Bewegbarkeit in einer Querrichtung ein eindimensionales (Linien-) Scannen ermöglicht, und durch eine Bewegbarkeit in zwei aufeinander senkrecht stehenden Querrichtungen ein zweidimensionalen (Flächen-) Scannen und somit eine rastermikroskopische bzw. -endoskopische Aufnahme. Schließlich wird dadurch, dass das Objektiv eine wellenlängenabhängige Blende umfasst, die für die erste Beleuchtungsstrahlung eine kleinere Öffnung hat als für die zweite Beleuchtungsstrahlung, eine konfokale Endoskopie mit besonders hoher Auflösung ermöglicht, ohne die Qualität der mit optischer Kohärenztomographie gewonnenen Tiefendaten zu beeinträchtigen.

Eine derartige kombinierte optische Kohärenztomographie und konfokale Endoskopie ermöglicht eine besonders vollständige und besonders genaue Erfassung von Gewebestrukturen im beobachteten Gewebebereich, wobei beispielsweise durch zweidimensionales Scannen mittels konfokaler Endoskopie Oberflächen- oder oberflächennahe Bilddaten gewonnen werden können, während durch ein- oder zweidimensionales Scannen mittels optischer Kohärenztomographie ein Längsschnitt durch den Gewebebereich bzw. ein dreidimensionale Erfassung von Gewebestrukturen bis in eine Tiefe von einigen Millimetern ermöglicht wird. Die Tiefenauflösung mittels OCT ermöglicht beispielsweise auch die Messung von Schichtdicken im Gewebe, die Aufschluss über eine mögliche Erkrankung des Gewebes geben können. Insbesondere wird eine gleichzeitige konfokale sowie optische kohärenztomographische Aufnahme gleicher Gewebebereiche zur Untersuchung von zellularen und strukturellen Gewebsveränderungen ermöglicht. Ebenso kann bei technischen Objekten eine simultane konfokale sowie optisch-kohärenztomographische Aufnahme gleicher Objektbereiche vorteilhaft sein.

Die endoskopische Sonde kann einen Schaft aufweisen, innerhalb dessen der Lichtleiter und das Objektiv aufgenommen sind. Der Schaft kann starr, halbstarr oder flexibel sein. Die Sonde bzw. der Schaft kann beispielsweise einen Durchmesser von 1 bis 3 mm haben. Die Sonde kann in einen Arbeitskanal eines Endoskops einführbar sein oder einen endoskopisch einsetzbaren Einführabschnitt umfassen oder beispielsweise als Katheter oder als Endoskopoptik ausgebildet sein.

Gemäß einer bevorzugten Ausführungsform der Erfindung ist die distale Endfläche der Lichtleitfaser selbstparallel bewegbar. Zum Scannen kann somit ein distaler Endabschnitt der Lichtleitfaser derart bewegt werden, dass die distale Endfläche lediglich in Querrichtung relativ zum Objektiv und zur optischen Achse verschoben wird und eine Senkrechte auf die Endfläche ihre Richtung unverändert beibehält und insbesondere stets zumindest näherungsweise parallel zur optischen Achse des Objektivs bleibt. Die Scanbewegung kann dabei ein- oder zweiachsig erfolgen. Zur Gewährleistung einer selbstparallelen Bewegung der distalen Endfläche kann ein distaler Endabschnitt der Lichtleitfaser in einer entsprechend geführten Halterung aufgenommen sein, die von einem Antrieb in Querrichtung bewegbar ist. Hierdurch wird eine besonders exakt steuerbare Scanbewegung ermöglicht.

Gemäß einer alternativen bevorzugten Ausführungsform der Erfindung ist ein distaler Endabschnitt der Lichtleitfaser abwinkelbar, beispielsweise in Querrichtung elastisch biegbar oder in einer Halterung aufgenommen, die um eine quer zur Längsrichtung der Lichtleitfaser stehende Achse schwenkbar ist. Beim Durchführen der Scanbewegung bildet somit die Senkrechte auf die distale Endfläche der Lichtleitfaser einen veränderlichen Winkel zur optischen Achse des Objektivs. Hierdurch kann auf besonders einfache Weise und mit einem besonders einfachen Antrieb die Scanbewegung realisiert werden, beispielsweise durch eine resonante Schwingbewegung. Auch in diesem Fall ist ein ein- oder zweiachsiges Scannen möglich.

Die Lichtleitfaser kann in vorteilhafter Weise als Monomodefaser oder als Photonikkristallfaser (Photonic Crystal Fiber, PCF) ausgebildet sein. Hierdurch wird die Übertragung der Beleuchtungs- und Beobachtungsstrahlung für die optische Kohärenztomographie sowie für die konfokale Endoskopie ermöglicht.

In besonders bevorzugter Weise ist die Lichtleitfaser oder ein Abschnitt der Lichtleitfaser als Doppelmantelfaser (Double-clad Fiber) ausgebildet. Eine Doppelmantelfaser umfasst einen Kern mit einem geringen Durchmesser, einen inneren Mantel und einen äußeren Mantel sowie ggf. eine Umhüllung. Der Kern ist in der Regel für eine Monomode (Single Mode) - Lichtweiterleitung ausgebildet, während der innere Mantel eine Multimode-Faser darstellt. Der Kern der Doppelmantelfaser kann somit zur Weiterleitung der ersten Beleuchtungsstrahlung und eines Anteils der Beobachtungsstrahlung unter weitgehender Erhaltung der Kohärenz genutzt werden, während der innere Mantel beispielsweise zur Weiterleitung eines weiteren Anteils der Beobachtungsstrahlung genutzt werden kann. Alternativ kann die Lichtleitfaser als Monomode-Faser ausgebildet sein.

Die Lichtleitfaser kann auch aus mehreren Faserabschnitten mit unterschiedlichen Fasertypen bestehen, die zusammen die einzelne Lichtleitfaser bilden. Es kann sich dabei um die zuvor genannten Fasertypen handeln. Es kann ein proximaler Bereich der Lichtleitfaser als Doppelmantelfaser ausgebildet sein und ein distaler Endabschnitt der Faser einen oder mehrere andere Fasertypen, wie beispielsweise eine Gradientenindexfaser, umfassen.

Vorzugsweise ist das Objektiv zum Einkoppeln zumindest eines durch die erste Beleuchtungsstrahlung beispielsweise durch Reflexion und/oder Streuung im untersuchten Gewebebereich erzeugten Anteils der Beobachtungsstrahlung in den Kern der Lichtleitfaser ausgebildet. Hierdurch kann insbesondere eine Weiterleitung dieses Anteils der Beobachtungsstrahlung zur proximalen Endfläche des Lichtleiters unter weitgehender Erhaltung der Kohärenz ermöglicht werden, wo die Beobachtungsstrahlung ausgekoppelt werden kann und für die Auswertung für die optische Kohärenztomographie zur Verfügung steht.

Weiter kann es vorgesehen sein, dass die zweite Beobachtungsstrahlung ebenfalls in den Kern der Doppelmantelfaser eingekoppelt wird und vom Objektiv auf den Gewebebereich eingestrahlt wird, und das Objektiv derart ausgebildet und angeordnet ist, dass der Anteil der Beobachtungsstrahlung, der aufgrund der zweiten Beobachtungsstrahlung entsteht, teilweise ebenfalls auf die Endfläche des Kerns fokussiert wird und durch den Kern zur proximalen Endfläche weitergeleitet wird, wo er zur Auswertung ausgekoppelt werden kann. Es kann dabei vorgesehen sein, dass auch ein durch den inneren Mantel weitergeleiteter Anteil der Beobachtungsstrahlung für die konfokale Endomikroskopie genutzt wird. Hierdurch ist eine besonders verlustarme Übertragung der zweiten Beleuchtungsstrahlung und der hierauf zurückgehenden Anteile der Beobachtungsstrahlung möglich.

Vorzugsweise umfasst die wellenlängenabhängige Blende ein ringförmiges Blendenelement, das im ersten Wellenlängenbereich im Wesentlichen undurchlässig und im zweiten Wellenlängenbereich im Wesentlichen durchlässig ist. Hierdurch wird erreicht, dass im ersten Wellenlängenbereich nur Licht innerhalb des inneren Randes des ringförmigen Blendenelements hindurchtreten kann, während im zweiten Wellenlängenbereich die gesamte Öffnung der Blende zur Verfügung steht. Somit kann die erste Beleuchtungsstrahlung mit einer geringen Apertur auf den Gewebebereich projiziert werden, so dass eine hohe Eindringtiefe und eine hohe Auflösung bei der optischen Kohärenztomographie erzielbar ist. Gleichzeitig kann die zweite Beleuchtungsstrahlung, die für die konfokale Endomikroskopie dient, mit einer hohen numerischen Apertur auf den Gewebebereich fokussiert werden, was die Erzielung einer hohen lateralen und axialen Auflösung ermöglicht. Das Verhältnis der numerischen Apertur für die zweite zu der für die erste Beleuchtungsstrahlung kann beispielsweise 2 oder mehr betragen.

In vorteilhafter Weise kann es vorgesehen sein, dass die wellenlängenabhängige Blende, insbesondere das ringförmige Blendenelement, schräg zu einer optischen Achse des Objektivs steht. Hierdurch kann das Auftreten von Reflexionen, die insbesondere bei der optischen Kohärenztomographie störend wären, weitgehend vermieden werden.

In besonders bevorzugter Weise kann das ringförmige Blendenelement der wellenlängenabhängigen Blende elliptisch ausgebildet sein. Insbesondere sind sowohl der innere Rand, der die Apertur im ersten Wellenlängenbereich definiert, als auch der äußere Rand, der die Apertur im zweiten Wellenlängenbereich bestimmt, elliptisch geformt, wobei die Hauptachsen der Ellipsen in einer Ebene liegen, die durch die Mittelsenkrechte des ringförmigen Blendenelements und die optische Achse des Objektivs aufgespannt wird. Bei einer entsprechenden Wahl der Exzentrizität der Ellipsen kann auf diese Weise erreicht werden, dass trotz der Schrägstellung der Blende in beiden Wellenlängenbereichen im Querschnitt jeweils kreisförmige Strahlenbündel durch die Blende hindurch treten.

Ein erfindungsgemäßes System für die endoskopische optische Kohärenztomographie und die konfokale Endoskopie ist insbesondere zur endoskopischen Aufnahme von Bilddaten eines Gewebebereichs eines menschlichen oder tierischen Körpers mittels kombinierter optischer Kohärenztomographie und konfokaler Endoskopie geeignet, beispielsweise von Bilddaten eines in einem körperinneren Hohlraum angeordneten Gewebebereichs. Das erfindungsgemäße System umfasst eine endoskopische Sonde, die wie zuvor beschrieben ausgebildet ist und die insbesondere in den körperinneren Hohlraum einführbar ist.

Weiterhin umfasst das erfindungsgemäße System eine Lichterzeugungseinrichtung, die zur Erzeugung der ersten Beleuchtungsstrahlung im ersten Wellenlängenbereich ausgebildet ist, die für eine optische Kohärenztomographie geeignet ist und insbesondere eine für die optische Kohärenztomographie geeignete Kohärenzlänge und einen hierfür ausreichenden Kohärenzgrad aufweist. Zur Erzeugung der ersten Beleuchtungsstrahlung kann die Lichterzeugungseinrichtung eine erste Lichtquelle umfassen, die beispielsweise eine Superlumineszenzdiode sein kann. Weiter ist die Lichterzeugungseinrichtung zur Erzeugung einer zweiten Beleuchtungsstrahlung in einem zweiten Wellenlängenbereich ausgebildet, die für eine konfokale Endomikroskopie geeignet ist und hierfür insbesondere eine hohe numerische Apertur aufweisen kann. Der erste und der zweite Wellenlängenbereich sind unterschiedliche Wellenlängenbereiche in den sichtbaren und/oder angrenzenden Bereichen des elektromagnetischen Spektrums, die sich vorzugsweise nicht überlappen. Die Lichterzeugungseinrichtung ist zum Einkoppeln der ersten und der zweiten Beleuchtungsstrahlung in die proximale Endfläche der Lichtleitfaser ausgebildet und angeordnet. In dem Fall, dass die Lichtleitfaser oder ein proximaler Abschnitt der Lichtleitfaser eine Doppelmantelfaser ist, insbesondere zum Einkoppeln der ersten Beleuchtungsstrahlung in den Kern der Doppelmantelfaser. Die Lichterzeugungseinrichtung kann neben den Lichtquellen dafür insbesondere weitere Lichtleiter, optische Bauteile und Faserkoppler umfassen.

Ferner umfasst das erfindungsgemäße System eine Detektoreinrichtung, die zur Erfassung von Bilddaten mittels optischer Kohärenztomographie ausgebildet ist. Hierfür kann die Detektoreinrichtung in an sich bekannter Weise ausgebildet sein und optische und optoelektronische Elemente umfassen, beispielsweise einen Photodetektor und geeignete Strahlführungs- und Koppelelemente. Die Detektoreinrichtung stellt insbesondere einen Teil einer Interferometeranordnung dar und kann einen Referenzarm umfassen. Dabei kann die Interferometeranordnung für eine Time-Domain-OCT vorgesehen sein, bei der die Länge des Referenzarms variabel ist, vorzugsweise ist die Anordnung jedoch für eine Spectral-Domain-OCT ausgebildet, bei der eine Interferenz bei unterschiedlichen Wellenlängen erfasst wird. Weiterhin ist die Detektoreinrichtung zur Erfassung von Bilddaten mittels konfokaler Endomikroskopie ausgebildet und umfasst hierfür insbesondere einen entsprechenden Detektor, beispielsweise ein CCD oder eine Photodiodenzeile, sowie ggf. weitere hierfür geeignete Strahlführungs- und Koppelelemente. Die Detektoranordnung ist ausgebildet und angeordnet, um aus der proximalen Endfläche der Lichtleitfaser austretende Beobachtungsstrahlung zur Erzeugung von Bilddaten mittels optischer Kohärenztomographie und konfokaler Endoskopie zu nutzen. Hierfür kann die Detektoranordnung beispielsweise einen dichroitischen Strahlteiler und/oder eine Filteranordnung aufweisen, der bzw. die einen Anteil der Beobachtungsstrahlung im ersten Wellenlängenbereich für die optische Kohärenztomographie zur Verfügung stellt und hierfür beispielsweise einer entsprechenden Interferometeranordnung zuführt, und einen weiteren Anteil der Beobachtungsstrahlung, insbesondere einen Anteil im zweiten Wellenlängenbereich, dem Detektor für die konfokale Endoskopie zuführt.

Weiter umfasst das erfindungsgemäße System eine Auswertungs- und Steuerungseinrichtung, die zum Steuern der Lichterzeugungseinrichtung, beispielsweise zur Ansteuerung einer oder mehrerer Lichtquellen der Lichterzeugungseinrichtung, und zum Steuern der Detektoreinrichtung, insbesondere zur Ansteuerung des Photodetektors für die optische Kohärenztomographie und des Detektors für die konfokale Endomikroskopie, sowie zum Steuern eines Antriebs des distalen Endabschnitts der Lichtleitfaser zum Scannen des Gewebebereichs ausgebildet und eingerichtet ist.

Das erfindungsgemäße System ermöglicht die Gewinnung von Bilddaten eines Objektbereichs, insbesondere eines Gewebebereichs eines menschlichen oder tierischen Gewebes, mittels optischer Kohärenztomographie und konfokaler Endoskopie mit einer besonders hohen Eindringtiefe und Auflösung, insbesondere eine endoskopische Erfassung von Bilddaten, und in besonders vorteilhafter Weise eine gleichzeitige Erfassung der optisch-kohärenztomographischen und der konfokalen Bilddaten.

Gemäß einer besonderen bevorzugten Ausführungsform der Erfindung sind die Lichterzeugungseinrichtung und die Detektoreinrichtung zur Aufnahme von Fluoreszenzdaten mittels der zweiten Beleuchtungsstrahlung ausgebildet; im Folgenden werden hierunter auch die Zwei-Photonen- oder Multiphotonen-Fluoreszenzanregung, ebenso wie andere nichtlineare Verfahren, etwa die Beobachtung höherer Harmonischer, verstanden. Dabei kann die Lichterzeugungseinrichtung zur Erzeugung einer Fluoreszenz- oder Multiphotonenanregungsstrahlung ausgebildet sein, die im zweiten Wellenlängenbereich liegt, und die Detektoreinrichtung zur Aufnahme von Bilddaten in einen dritten Wellenlängenbereich ausgebildet sein, der einer durch die zweite Beleuchtungsstrahlung erzeugten Fluoreszenzstrahlung entspricht. Ebenso können höhere Harmonische in dem dritten Wellenlängenbereich beobachtet werden. Da für derartige Beobachtungsmoden eine sehr hohe Energiedichte innerhalb des zu untersuchenden Gewebebereichs erzeugt werden muss, ist auch hierfür die Einstrahlung der zweiten Beleuchtungsstrahlung mit einer hohen numerischen Apertur besonders vorteilhaft. Als Lichtquelle kann beispielsweise eine Laserlichtquelle dienen.

In dem Fall, dass die Lichtleitfaser oder ein proximaler Abschnitt der Lichtleitfaser als Doppelmantelfaser ausgebildet ist, ist die Lichterzeugungseinrichtung vorzugsweise zum Einkoppeln der ersten Beleuchtungsstrahlung in den Kern der Doppelmantelfaser ausgebildet und angeordnet. Weiter kann die Detektoreinrichtung zum Aufteilen der aus dem Kern und/oder dem inneren Mantel der Lichtleitfaser austretenden Beobachtungsstrahlung nach Wellenlängenbereichen auf die Detektoren für die Kohärenztomographie und die konfokale Endoskopie ausgebildet sein. Hierdurch kann eine jeweils optimale Weiterleitung und Nutzung der für die optische Kohärenztomographie und der für die konfokale Endoskopie genutzten Strahlung ermöglicht werden.

Insbesondere in dem Fall, dass die zweite Beleuchtungsstrahlung zur Fluoreszenzanregung dient, kann in besonders vorteilhafter Weise eine Einkopplung der vom Gewebebereich abgegebenen Fluoreszenzstrahlung in den inneren Mantel der Doppelmantelfaser und eine Weiterleitung der am proximalen Ende der Lichtleitfaser aus dem inneren Mantel austretenden Beobachtungsstrahlung zur Detektoreinrichtung für die konfokale Endoskopie vorgesehen sein. Diese kann in diesem Fall beispielsweise einen weiteren Strahlteiler aufweisen, der einen Anteil der Beobachtungsstrahlung, der im zweiten Wellenlängenbereich liegt, zu einem ersten Detektor, und einen Anteil in einem hiervon verschiedenen Wellenlängenbereich zu einem Fluoreszenz- bzw. Multiphotonendetektor weiterleitet. Hierdurch werden weitere Beobachtungsmöglichkeiten geschaffen, die eine besonders vollständige Erfassung von Gewebestrukturen und deren Veränderungen ermöglichen.

Die Steuerungs- und Auswertungseinrichtung kann in vorteilhafter Weise auch zum Erzeugen eines oder mehrerer Bilder des beobachteten Gewebebereichs aufgrund der kombinierten kohärenztomographischen und der konfokalen Bilddaten eingerichtet sein. Dabei kann insbesondere aus den mittels optischer Kohärenztomographie aufgenommenen Bilddaten ein in Richtung der optischen Achse des Objektivs gerichtetes Längsschnittbild des Gewebebereichs erzeugt werden und aus den mittels konfokaler Endomikroskopie aufgenommenen Bilddaten ein Oberflächenbild des Gewebebereichs oder ein Querschnittsbild in geringer Tiefe des Gewebebereichs. Die erzeugten Bilder können auf einer entsprechenden Anzeigeeinrichtung dargestellt werden. Hierdurch ist eine simultane Aufnahme und Visualisierung molekularer, chemischer und zellularer sowie struktureller Gewebeveränderungen möglich. Ebenso kann eine solche Aufnahme und Visualisierung von Strukturen aufgrund der kombinierten kohärenztomographischen und konfokalen Bilddaten bei der Untersuchung nicht-medizinischer Objekte vorteilhaft sein.

Gemäß einem erfindungsgemäßen Verfahren wird eine kombinierte optische Kohärenztomographie und konfokale Endoskopie durchgeführt, d.h. es werden mit optischer Kohärenztomographie und mit konfokaler Endoskopie Bilddaten desselben Objektbereichs aufgenommen. Der Objektbereich kann insbesondere ein Bereich eines menschlichen oder tierischen Gewebes in einem körperinneren Hohlraum sein, das erfindungsgemäße Verfahren kann jedoch auch für nicht-medizinische Zwecke angewendet werden, beispielsweise zur Untersuchung von insbesondere teiltransparenten technischen Objekten.

Bei dem erfindungsgemäßen Verfahren werden in einem ersten Wellenlängenbereich eine erste Beleuchtungsstrahlung, die für die optisch-kohärenztomographische Gewinnung von Bilddaten geeignet ist und insbesondere eine entsprechende Kohärenz aufweist, und in einem zweiten Wellenlängenbereich eine zweite Beleuchtungsstrahlung, die für die konfokale Endoskopie geeignet ist und beispielsweise eine ausreichende Energiedichte und numerische Apertur aufweist, erzeugt. Die erste und die zweite Beleuchtungsstrahlung werden in eine proximale Endfläche eines Lichtleiters eingekoppelt, durch den Lichtleiter zu dessen distaler Endfläche weitergeleitet und treten aus einer distalen Endfläche des Lichtleiters aus. Die dort austretende erste und zweite Beleuchtungsstrahlung werden mit einem Objektiv auf einen zu beobachtenden Objektbereich eingestrahlt, insbesondere in eine Fokusebene fokussiert, in der das zu untersuchende Messobjekt, beispielsweise ein Gewebebereich eines menschlichen oder tierischen Körper, angeordnet ist.

Weiter wird eine vom Messobjekt, etwa dem Gewebebereich, ausgehende Beobachtungsstrahlung von dem Objektiv aufgenommen und auf die distale Endfläche des Lichtleiters fokussiert und dadurch in diesen eingekoppelt. Die Beobachtungsstrahlung wird durch den Lichtleiter zu dessen proximaler Endfläche weitergeleitet und aus der proximalen Endfläche des Lichtleiters ausgekoppelt. Die ausgekoppelte Beobachtungsstrahlung wird einer Detektoreinrichtung zugeführt, wobei ein Anteil der Beobachtungsstrahlung im ersten Wellenlängenbereich zur Erzeugung von optisch-kohärenztomographischen Bilddaten ausgewertet wird und ein weiterer Anteil der Beobachtungsstrahlung zur Erzeugung von konfokalen Bilddaten ausgewertet wird.

Erfindungsgemäß ist der Lichtleiter als eine einzelne Lichtleitfaser ausgebildet, und die distale Endfläche des Lichtleiters bzw. der einzelnen Lichtleitfaser wird zum Scannen des Messobjekts bzw. des Gewebebereichs quer zur optischen Achse des Objektivs bewegt. Das erfindungsgemäße Verfahren ist insbesondere ein Verfahren zum Betreiben eines wie oben beschrieben ausgebildeten erfindungsgemäßen Systems für endoskopische optische Kohärenztomographie und konfokale Endoskopie.

Mit dem erfindungsgemäßen Verfahren ist sowohl eine besonders hohe laterale und axiale Auflösung bei der Aufnahme endomikroskopischer Bilddaten, als auch eine besonders hohe Eindringtiefe bei der optisch-kohärenztomographischen Datenaufnahme erreichbar. Insbesondere kann eine gleichzeitige Durchführung der optischen Kohärenztomographie und der konfokalen Endoskopie zur Untersuchung eines Objektbereichs ermöglicht werden. Ferner kann das erfindungsgemäße Verfahren, insbesondere zur Untersuchung eines Gewebebereichs eines menschlichen oder tierischen Gewebes in einem körperinneren Hohlraum, mittels einer in einen Arbeitskanal eines Endoskops eingeführten Sonde durchgeführt werden. Hierdurch wird eine besonders vollständige und besonders genaue Erfassung von Gewebestrukturen im untersuchten Gewebebereich zur Detektion von zellularen und strukturellen Gewebsveränderungen ermöglicht.

In dem Fall, dass die Lichtleitfaser oder ein proximaler Abschnitt der Lichtleitfaser als Doppelmantelfaser ausgebildet ist, kann das erfindungsgemäße System in besonders vorteilhafter Weise derart betrieben werden, dass die erste Beleuchtungsstrahlung an der proximalen Endfläche in den Kern der Doppelmantelfaser eingekoppelt wird, durch den Kern der Doppelmantelfaser zur distalen Endfläche weitergeleitet wird, dort aus der distalen Endfläche austritt und vom Objektiv auf den zu untersuchenden Objekt- bzw. Gewebebereich eingestrahlt wird. Ebenso wird der Anteil der Beobachtungsstrahlung, der durch Reflexion oder Streuung der ersten Beleuchtungsstrahlung innerhalb des Objekts bzw. im Gewebe entsteht, vom Objektiv zumindest teilweise ebenfalls auf die Endfläche des Kerns fokussiert und dadurch in den Kern eingekoppelt und durch den Kern unter weitgehender Erhaltung der Kohärenz zur distalen Endfläche weitergeleitet, wo er zur Auswertung ausgekoppelt werden kann. In weiter vorteilhafter Weise kann auch die zweite Beleuchtungsstrahlung am proximalen Ende der als Doppelmantelfaser ausgebildeten Lichtleitfaser in deren Kern eingekoppelt und der auf die zweite Beleuchtungsstrahlung zurückgehende Anteil der Beobachtungsstrahlung vom Objektiv zum Teil ebenfalls in den Kern eingekoppelt werden. Es kann dabei vorgesehen sein, dass auch ein durch den inneren Mantel weitergeleiteter Anteil der Beobachtungsstrahlung für die konfokale Endomikroskopie benutzt wird. Hierdurch ist eine besonders verlustarme Übertragung der ersten sowie gegebenenfalls der zweiten Beleuchtungsstrahlung und der hierauf zurückgehenden Anteile der Beobachtungsstrahlung möglich. Dies gilt insbesondere, wenn die zweite Beleuchtungsstrahlung zur Fluoreszenz- oder Multiphotonenanregung genutzt wird und entsprechende Bilddaten gewonnen werden.

Vorzugsweise wird die distale Endfläche der Lichtleitfaser in zwei Richtungen quer zur optischen Achse des Objektivs bewegt, um das Messobjekt bzw. den Gewebebereich zweidimensional zu scannen. Aus den optisch-kohärenztomographischen Bilddaten werden in diesem Fall 3D-Daten des Objekt- bzw. Gewebebereichs erzeugt und/oder es wird aus den konfokalen Bilddaten ein zweidimensionales oberflächennahes Bild des Objekt- bzw. Gewebebereichs erzeugt.

Es versteht sich, dass die vorstehend genannten und die nachstehend noch zu erläuternden Merkmale nicht nur in der jeweils angegebenen Kombination, sondern auch in anderen Kombinationen verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 ein Ausführungsbeispiel eines erfindungsgemäßen Systems für endoskopische optische Kohärenztomographie und konfokale Endoskopie;
Fig. 2a bis 2c den distalen Endbereich der in dem System gemäß Fig. 1 eingesetzten Sonde für optische Kohärenztomographie und konfokale Endoskopie in einem Längsschnitt sowie die Doppelmantelfaser und die wellenlängenabhängige Blende der Sonde in einem Querschnitt bzw. in einer axialen Ansicht;
Fig. 3 den distalen Endbereich der Sonde wie in Fig. 2a, wobei der distale Endabschnitt der Doppelmantelfaser gemäß einer ersten Ausführungsform der Erfindung ausgelenkt ist;
Fig. 4 den distalen Endbereich der Sonde wie in Fig. 2a, wobei der distale Endabschnitt der Doppelmantelfaser gemäß einer zweiten Ausführungsform der Erfindung ausgelenkt ist.

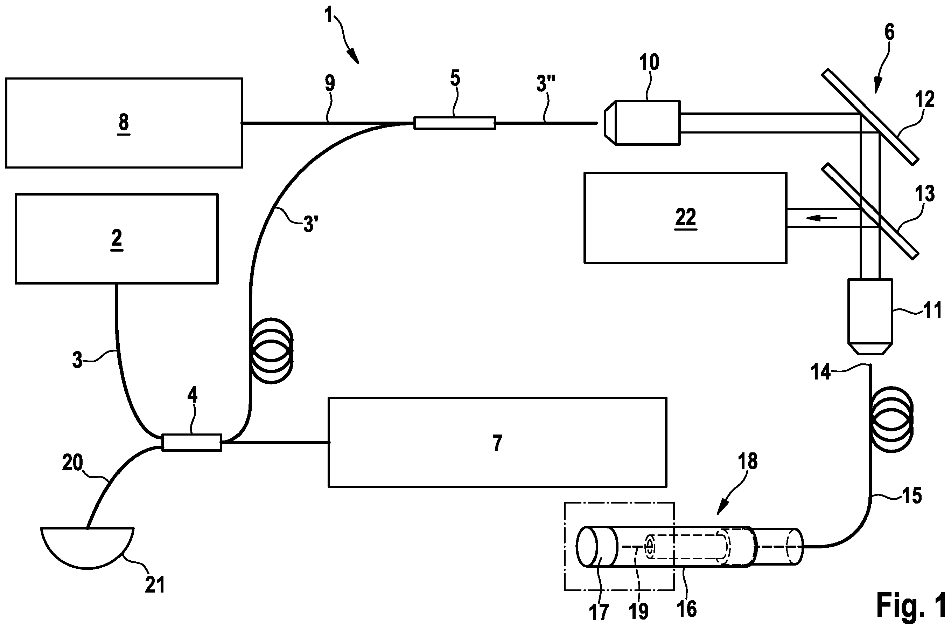

In Fig. 1 ist ein erfindungsgemäßes System 1 zur kombinierten optischen Kohärenztomographie und konfokalen Endoskopie beispielhaft in vereinfachter Form dargestellt. Das System 1 umfasst eine OCT-Lichtquelle 2 für die optische Kohärenztomographie, die beispielsweise eine Superlumineszenzdiode sein kann. Die von der OCT-Lichtquelle 2 erzeugte teilweise kohärente OCT-Beleuchtungsstrahlung wird in einen Lichtleiter 3 eingekoppelt; ein Anteil der Beleuchtungsstrahlung gelangt über einen ersten und einen zweiten Faserkoppler 4, 5 und weitere Lichtleiterabschnitte 3', 3 "zu einer Strahlteileroptik 6. Ein weiterer Anteil der von der OCT-Lichtquelle 2 erzeugten Beleuchtungsstrahlung wird im ersten Faserkoppler 4 ausgekoppelt und in einen Referenzarm 7 geleitet und gelangt durch diesen zurück zum ersten Faserkoppler 4. Die optische Weglänge des Referenzarms 7 entspricht näherungsweise der optischen Weglänge des Messarms vom ersten Faserkoppler 4 zu einem beobachteten Gewebebereich und von diesem zurück zum ersten Faserkoppler 4 (s.u.).

Das System 1 umfasst weiterhin eine Lichtquelle für die konfokale Endoskopie, die im gezeigten Ausführungsbeispiel eine Laserlichtquelle 8 für eine Fluoreszenzanregung ist. Die von der Laserlichtquelle 8 erzeugte Beleuchtungsstrahlung wird in einen Lichtleiter 9 eingekoppelt und gelangt über den zweiten Faserkoppler 5 und den Lichtleiterabschnitt 3" ebenfalls zur Strahlteileroptik 6. Die Strahlteileroptik 6 ist in Fig. 1 symbolisch mit zwei Koppeloptiken 10, 11, einem Umlenkspiegel 12 und einem dichroitischen Strahlteiler 13 dargestellt. Die Beleuchtungsstrahlung, die die von der OCT-Lichtquelle 2 erzeugte OCT-Beleuchtungsstrahlung und die von der Laserlichtquelle erzeugte Fluoreszenzanregungsstrahlung enthält, tritt durch den dichroitischen Strahlteiler 13 hindurch und wird von der Koppeloptik 11 in die proximale Endfläche 14 einer einzelnen Lichtleitfaser, der Doppelmantelfaser 15, und dort in den Kern der Doppelmantelfaser 15 eingekoppelt. Die Lichtquellen, Fasern, Faserkoppler und optischen Bauteile proximal der proximalen Endfläche 14 sind Teil der Lichterzeugungseinrichtung.

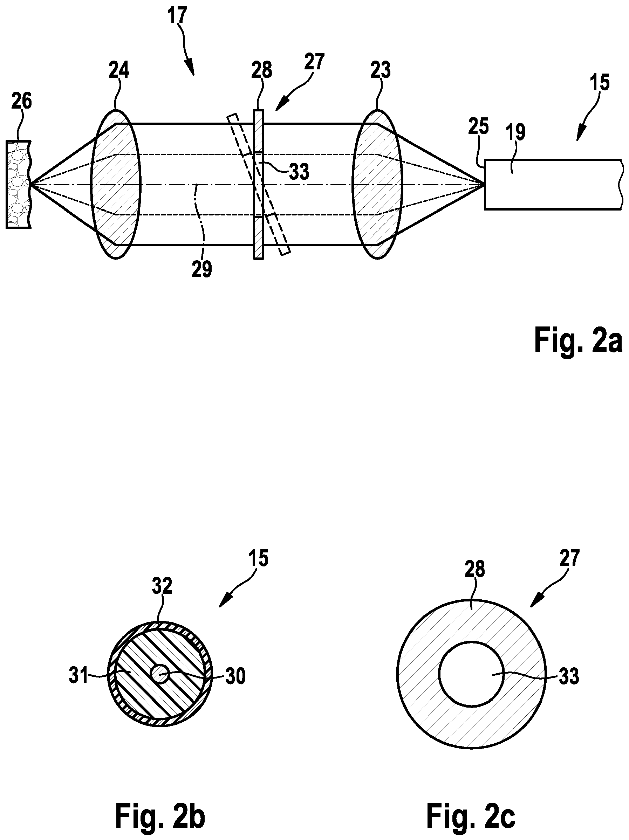

Ein distaler Bereich der Doppelmantelfaser 15 ist in einem Schaft 16 aufgenommen, der für die Einführung in einen Arbeitskanal eines Endoskops geeignet ist. Innerhalb des Schafts 16 ist ferner in einer distalseitigen axialen Verlängerung der Doppelmantelfaser 15 ein Objektiv 17 aufgenommen, das in Bezug auf eine Mittelstellung des distalen Endabschnitts 19 der Lichtleitfaser in einer geraden Verlängerung des distalen Endabschnitts 19 angeordnet ist. Der Schaft 16 mit den in diesem angeordneten Bauteilen und die Doppelmantelfaser 15 bilden eine endoskopische Sonde 18. Der in Fig. 1 mit einem Rechteck markierte Bereich der Sonde ist in den Figuren 2a bis 4 vergrößert dargestellt (s. unten).

Das durch den Kern der Doppelmantelfaser 15 weitergeleitete Beleuchtungslicht tritt distalseitig aus der Doppelmantelfaser 15 aus und wird vom Objektiv 17 auf ein in Fig. 1 nicht dargestelltes Messobjekt, beispielsweise einen Gewebebereich, eingestrahlt. Vom Messobjekt zurückkommendes Licht wird vom Objektiv 17 aufgenommen und in die Doppelmantelfaser 15 eingekoppelt. Diese, von der Doppelmantelfaser 15 in proximaler Richtung weitergeleitete Beobachtungsstrahlung tritt aus der proximalen Endfläche 14 der Doppelmantelfaser 15 aus und gelangt über die Koppeloptik 11 in die Strahlteileroptik 6. Der dichroitische Strahlteiler 13 lässt denjenigen Anteil der Beobachtungsstrahlung, der in dem Wellenlängenbereich der von der OCT-Lichtquelle 2 erzeugten Beleuchtungsstrahlung liegt, hindurchtreten. Dieser Anteil gelangt über den Umlenkspiegel 12, die Koppeloptik 10, den Lichtleiterabschnitt 3", den zweiten Faserkoppler 5 und den Lichtleiterabschnitt 3' zum ersten Faserkoppler 4. Dort tritt dieser Anteil der Beobachtungsstrahlung in Interferenz mit dem aus dem Referenzarm 7 zurückkommenden Anteil der von der OCT-Lichtquelle 2 erzeugten OCT-Beleuchtungsstrahlung. Das hierbei entstehende Interferenzsignal wird über einen Lichtleiter 20 zu einer Detektoreinrichtung geleitet, die hier einen Photodetektor 21 umfasst, wo es aufgenommen wird und zur weiteren Auswertung zur Verfügung steht.

Weitere Anteile der aus der proximalen Endfläche 14 der Doppelmantelfaser 15 austretenden Beobachtungsstrahlung werden vom dichroitischen Strahlteiler 13 zu einem Fluoreszenzdetektor 22 reflektiert, der Teil der Detektoreinrichtung ist, wo die vom Gewebe emittierte Fluoreszenzstrahlung detektiert wird. Hierfür ist der dichroitische Strahlteiler 13 im Wellenlängenbereich der Fluoreszenzstrahlung reflektierend ausgebildet. Der dichroitische Strahlteiler 13 kann auch im Wellenlängenbereich der von der Laserlichtquelle 8 erzeugten Fluoreszenzanregungsstrahlung reflektierend ausgebildet sein; in diesem Fall kann vom Fluoreszenzdetektor auch ein vom Gewebe zurückreflektierter Anteil der Fluoreszenzanregungsstrahlung detektiert werden.

In Fig. 2a ist der in Fig. 1 mit einem Rechteck markierte Ausschnitt vergrößert gezeigt. Das Objektiv 17 ist dabei vereinfacht mit einer proximalen Linse 23 und einer distalen Linse 24 dargestellt. Das aus der distalen Endfläche 25 der Doppelmantelfaser 15 austretende Beleuchtungslicht wird von den Linsen 23, 24 auf einen zu untersuchenden Gewebebereich 26 fokussiert. In der Nähe einer Hauptebene des Objektivs 17, in dem in Fig. 2a gezeigten Beispiel etwa in der Mitte zwischen den beiden Linsen 23, 24, ist eine wellenlängenabhängig wirkende kreisförmige Blende 27 angeordnet, die ein kreisringförmiges Blendenelement 28 umfasst. Das Blendenelement 28 ist im Wellenlängenbereich der von der OCT-Lichtquelle erzeugten Beleuchtungsstrahlung undurchlässig, jedoch im Wellenlängenbereich der Fluoreszenzanregungsstrahlung und insbesondere im Wellenlängenbereich der von dem Gewebe 26 emittierten Fluoreszenzstrahlung durchlässig. Gemäß einer in Fig. 2a ebenfalls angedeuteten Variante kann die Blende 27 relativ zur optischen Achse 29 des Objektivs 17 schräg gestellt sein; in diesem Fall ist das Blendenelement 28 elliptisch ausgebildet.

In den Figuren 2b und 2c sind die Doppelmantelfaser 15 im Querschnitt und die Blende 27 in einer axialen Draufsicht dargestellt. Wie in Fig. 2b gezeigt ist, umfasst die Doppelmantelfaser 15 einen Kern 30, einen inneren Mantel 31 und einen äußeren Mantel 32. Der Kern 30 wirkt als Monomodefaser und ist zur Kohärenz erhaltenden Weiterleitung der Beleuchtungs- und der Beobachtungsstrahlung geeignet. Der Kern der Doppelmantelfaser hat einen Durchmesser von wenigen Mikrometern, beispielsweise ca. 9 µm, während der innere Mantel der Doppelmantelfaser 15 einen Durchmesser von ca. 100 µm hat. Wie in Fig. 2c gezeigt ist, weist die Blende 27 ein ringförmiges Blendenelement 28 mit einer freien Öffnung 33 auf.

Die aus der distalen Endfläche 25 mit einer hohen numerischen Apertur austretende Fluoreszenzanregungsstrahlung wird durch das kreisringförmige Blendenelement 28 hindurchgelassen und mit einer hohen numerischen Apertur auf das Gewebe 26 fokussiert. In Fig. 2a sind die Randstrahlen dieses Strahlenbündels als durchgezogene Linien dargestellt. Ebenso gelangt aus dem Gewebe 26 zurückkommende reflektierte Beleuchtungsstrahlung, wie auch die durch Fluoreszenzstoffe im Gewebe 26 erzeugte Fluoreszenzstrahlung, mit einer hohen numerischen Apertur durch die Blende 27 hindurch und wird mit einer hohen numerischen Apertur auf die distale Endfläche 25 der Doppelmantelfaser abgebildet; die rückkommenden Strahlen verlaufen wie die Strahlen der Fluoreszenzanregungsstrahlung, die in Fig. 2a als durchgezogene Linien dargestellt sind. Vom Objektiv 17 aufgenommene Lichtanteile, die nicht auf den Kern 30 der Doppelmantelfaser 15, sondern auf den inneren Mantel 31 abgebildet werden, werden durch den inneren Mantel 31 zur proximalen Endfläche 14 weitergeleitet, wo sie ebenfalls für die Detektion der Fluoreszenz genutzt werden können.

Andererseits wird die aus dem Kern 30 der Doppelmantelfaser 15 an der distalen Endfläche 25 austretende OCT-Beleuchtungsstrahlung, die ebenfalls eine hohe numerische Apertur haben kann, durch das ringförmige Blendenelement 28 auf eine geringe numerische Apertur begrenzt, die der Öffnung 33 entspricht und die beispielsweise etwa die Hälfte der numerischen Apertur der Fluoreszenzanregungsstrahlung sein kann. Die OCT-Beleuchtungsstrahlung wird somit mit einer geringen numerischen Apertur auf das Gewebe 26 eingestrahlt, und vom Gewebe 26 zurückreflektierte Strahlung in demselben Wellenlängenbereich wird ebenfalls nur mit einer geringen numerischen Apertur in den Kern 30 der Lichtleitfaser zur Weiterleitung in proximaler Richtung eingekoppelt. Die Randstrahlen dieses Strahlenbündels sind in Fig. 2a als gestrichelte Linien dargestellt. Auf diese Weise wird es erreicht, dass sowohl für die konfokale bzw. Fluoreszenz-Endoskopie als auch für die optisch-kohärenztomographische Gewinnung von Tiefendaten des Gewebebereichs 26 eine jeweils optimale Apertur genutzt werden kann.

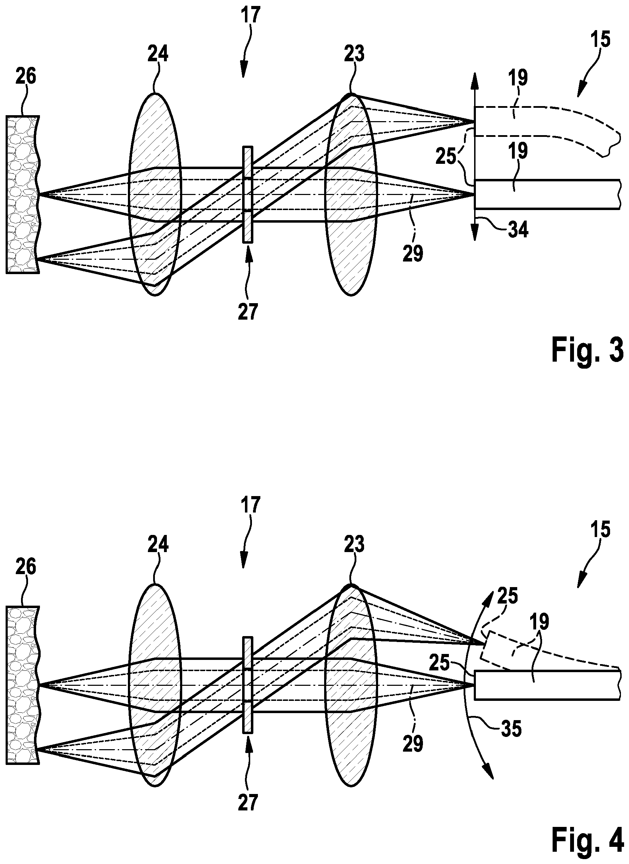

Um aus den wie zuvor beschrieben punktweise erfassten Bilddaten ein mehrdimensionales Bild des Gewebebereichs 26 zu erzeugen, wird die distale Endfläche 25 der Doppelmantelfaser quer zur optischen Achse 29 des Objektivs 17 bewegt. Dies ist für zwei unterschiedliche Ausführungsformen schematisch in den Figuren 3 und 4 dargestellt.

Wie in Fig. 3 gezeigt, wird gemäß einer ersten Ausführungsform der distale Endabschnitt 19 der Doppelmantelfaser 15 zu sich selbst parallel in einer Richtung quer zur optischen Achse 29 des Objektivs 17 bewegt, wobei eine Senkrechte auf die distale Endfläche parallel zur optischen Achse 29 bleibt. In Fig. 3 sind eine entsprechend ausgelenkte Position und die Mittelstellung des distalen Endabschnitts 19 gezeigt. Zur Ausführung der hin- und hergehenden Auslenkungsbewegung in ein oder zwei Achsen ist in der Sonde 18 ein hierfür geeigneter Antrieb vorgesehen, etwa ein piezomotorischer Antrieb. Für die numerische Apertur und die Einkopplung der vom beleuchteten Punkt des Gewebebereichs 26 zurückkommenden Strahlung gilt das Gleiche wie zuvor beschrieben, wobei ebenfalls die Randstrahlen des Strahlenbündels der Fluoreszenzanregungs- und Fluoreszenzstrahlung als durchgezogene Linien und die Randstrahlen des für die Kohärenztomographie genutzten Strahlenbündels als gestrichelte Linien dargestellt sind; außerdem ist ein mittlerer Strahl der Strahlenbündel angedeutet. Dadurch, dass die distale Endfläche 25 quer zur optischen Achse 29 bewegt wird, werden sowohl die Beleuchtungsstrahlung für die optische Kohärenztomographie als auch die Fluoreszenzanregungsstrahlung auf gegenüber der optischen Achse 29 versetzte Objektpunkte des Gewebebereichs 26 eingestrahlt. Hierdurch kann die Oberfläche des Gewebebereichs 26 in ein oder zwei Dimensionen abgetastet (gescannt) werden. Die Bewegung der distalen Endfläche 25 ist in Fig. 3 durch den Pfeil 34 angedeutet.

Bei der in Fig. 4 gezeigten Ausführungsform erfolgt ein Scannen der Oberfläche des Gewebebereichs 26 in entsprechender Weise, wobei jedoch der distale Endabschnitt 19 in einer Richtung quer zur optischen Achse 29 des Objektivs 17 abgewinkelt wird. Die Scanbewegung ist in Fig. 4 durch den gekrümmten Pfeil 35 angedeutet. Im Übrigen gilt das Gleiche wie zu Fig. 3 erläutert. In den Fig. 2a, 3 und 4 ist die Einstrahlung der Beleuchtungsstrahlung und die Emission der zurückkommenden Strahlung jeweils vereinfacht in Bezug auf einen Objektpunkt an der Oberfläche des Gewebebereichs dargestellt. Insbesondere die Kohärenztomographie ermöglicht eine Erfassung von Tiefendaten bis zu einer Tiefe von einigen Millimetern, beispielsweise 1 bis 2 mm, unter der Oberfläche des Gewebebereichs 26.

Das System 1 umfasst auch eine Auswertungs- und Steuerungseinrichtung, die zum Ansteuern der OCT-Lichtquelle 2, der Laserlichtquelle 8, des Photodetektors 21 und des Fluoreszenzdetektors 22 sowie zum Steuern eines Antriebs des distalen Endabschnitts 19 der Lichtleitfaser zum Scannen des Gewebebereichs und zum Auswerten der Signale des Photodetektors 21 und des Fluoreszenzdetektors 22 zum Erzeugen von Bildern bzw. 3D-Darstellungen des Gewebebereichs 26 ausgebildet und eingerichtet ist. Die Auswertungs- und Steuerungseinrichtung ist in den Figuren nicht dargestellt. Die Erfindung ist für die Untersuchung eines Gewebebereichs 26 eines menschlichen oder tierischen Gewebes beschrieben worden, ist in entsprechender Weise aber auch für die Untersuchung beispielsweise eines Bereichs eines technischen Objekts anwendbar.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: System
- 2: OCT-Lichtquelle
- 3: Lichtleiter
- 3', 3": Lichtleiterabschnitt
- 4: Faserkoppler
- 5: Faserkoppler
- 6: Strahlteileroptik
- 7: Referenzarm
- 8: Laserlichtquelle
- 9: Lichtleiter
- 10: Koppeloptik
- 11: Koppeloptik
- 12: Umlenkspiegel
- 13: Strahlteiler
- 14: Proximale Endfläche
- 15: Doppelmantelfaser
- 16: Schaft
- 17: Objektiv
- 18: Sonde
- 19: Distaler Endabschnitt
- 20: Lichtleiter
- 21: Photodetektor
- 22: Fluoreszenzdetektor
- 23: Linse
- 24: Linse
- 25: Distale Endfläche
- 26: Gewebebereich
- 27: Blende
- 28: Blendenelement
- 29: Optische Achse
- 30: Kern
- 31: Innerer Mantel
- 32: Äußerer Mantel
- 33: Öffnung
- 34: Pfeil
- 35: Pfeil

## Patentansprüche

1. Endoskopische Sonde für optische Kohärenztomographie und konfokale Endoskopie, umfassend einen langerstreckten Lichtleiter zum Weiterleiten einer ersten Beleuchtungsstrahlung für die Kohärenztomographie, die einen ersten Wellenlängenbereich umfasst, und einer zweiten Beleuchtungsstrahlung für die konfokale Endoskopie, die einen zweiten Wellenlängenbereich umfasst, von einer proximalen Endfläche (14) zu einer distalen Endfläche (25) des Lichtleiters, sowie ein Objektiv (17) zum Weiterleiten der ersten und der zweiten Beleuchtungsstrahlung von der distalen Endfläche (25) des Lichtleiters zu einem zu beobachtenden Objektbereich, insbesondere eines Gewebebereichs (26) eines menschlichen oder tierischen Gewebes, und zum Weiterleiten einer von dem Objektbereich ausgehenden Beobachtungsstrahlung zur distalen Endfläche (25) des Lichtleiters, wobei das Objektiv (17) eine wellenlängenabhängige Blende (27) umfasst, die im ersten Wellenlängenbereich eine kleinere Blendenöffnung hat als im zweiten Wellenlängenbereich, und wobei der Lichtleiter ferner zur Weiterleitung der eingekoppelten Beobachtungsstrahlung zur proximalen Endfläche (14) des Lichtleiters ausgebildet ist, **dadurch gekennzeichnet, dass** der Lichtleiter als eine einzelne Lichtleitfaser ausgebildet ist und dass die distale Endfläche (25) der Lichtleitfaser quer zur optischen Achse (29) des Objektivs (17) bewegbar ist.

2. Endoskopische Sonde nach Anspruch 1, **dadurch gekennzeichnet, dass** die distale Endfläche (25) der Lichtleitfaser selbstparallel bewegbar ist.

3. Endoskopische Sonde nach Anspruch 1, **dadurch gekennzeichnet, dass** ein distaler Endabschnitt (19) der Lichtleitfaser abwinkelbar ist.

4. Endoskopische Sonde nach einem der vorhergehenden Ansprüche, **dadurch gekennzeichnet, dass** die Lichtleitfaser oder ein Abschnitt der Lichtleitfaser als Doppelmantelfaser (15) ausgebildet ist.

5. Endoskopische Sonde nach Anspruch 4, **dadurch gekennzeichnet, dass** das Objektiv (17) zum Einkoppeln zumindest eines durch die erste Beleuchtungsstrahlung erzeugten Anteils der Beobachtungsstrahlung in einen Kern (30) der Doppelmantelfaser (15) ausgebildet ist.

6. Endoskopische Sonde nach einem der vorhergehenden Ansprüche, **dadurch gekennzeichnet, dass** die wellenlängenabhängige Blende (27) ein ringförmiges Blendenelement (28) umfasst, das im ersten Wellenlängenbereich im Wesentlichen undurchlässig und im zweiten Wellenlängenbereich im Wesentlichen durchlässig ist.

7. Endoskopische Sonde nach einem der vorhergehenden Ansprüche, **dadurch gekennzeichnet, dass** die wellenlängenabhängige Blende (27) schräg zur optischen Achse des Objektivs steht.

8. Endoskopische Sonde nach Anspruch 6 in Kombination mit Anspruch 7, **dadurch gekennzeichnet, dass** das ringförmige Blendenelement (28) der wellenlängenabhängigen Blende (27) elliptisch ausgebildet ist.

9. System für endoskopische optische Kohärenztomographie (OCT) und konfokale Endoskopie, umfassend eine endoskopische Sonde (18) nach einem der vorhergehenden Ansprüche, eine Lichterzeugungseinrichtung zur Erzeugung der ersten und der zweiten Beleuchtungsstrahlung, eine Detektoreinrichtung (20, 21), die zur Erfassung von OCT-Bilddaten aufgrund eines durch die erste Beleuchtungsstrahlung erzeugten Anteils der Beobachtungsstrahlung und von endomikroskopischen Bilddaten aufgrund eines durch die zweite Beleuchtungsstrahlung erzeugten Anteils der Beobachtungsstrahlung ausgebildet ist, und eine Auswertungs- und Steuerungseinrichtung, die zum Steuern der Lichterzeugungseinrichtung und der Detektoreinrichtung (20, 21) und zum Steuern eines Antriebs des distalen Endabschnitts (19) der Lichtleitfaser zum Scannen des Objektbereichs (26) ausgebildet und eingerichtet ist.

10. System nach Anspruch 9, **dadurch gekennzeichnet, dass** die Lichterzeugungseinrichtung und die Detektoreinrichtung (20, 21) zur Erzeugung von Fluoreszenz- und/oder Multiphotonenbilddaten mittels der zweiten Beleuchtungsstrahlung ausgebildet sind.

11. System nach Anspruch 9 oder 10, **dadurch gekennzeichnet, dass** die Lichtleitfaser oder ein proximaler Abschnitt der Lichtleitfaser als Doppelmantelfaser (15) ausgebildet ist und die Lichterzeugungseinrichtung zum Einkoppeln der ersten Beleuchtungsstrahlung in den Kern (30) der Doppelmantelfaser (15) ausgebildet und angeordnet ist.

12. System nach einem der Ansprüche 9 bis 11, **dadurch gekennzeichnet, dass** die Auswertungs- und Steuerungseinrichtung zum Erzeugen eines oder mehrerer Bilder und/oder 3D-Daten des Objektbereichs aufgrund der erfassten Bilddaten ausgebildet ist.

13. Verfahren zur kombinierten optischen Kohärenztomographie und konfokalen Endoskopie, wobei in einem ersten Wellenlängenbereich eine erste Beleuchtungsstrahlung für die Kohärenztomographie und in einem zweiten Wellenlängenbereich eine zweite Beleuchtungsstrahlung für die konfokale Endoskopie erzeugt werden, die erste und die zweite Beleuchtungsstrahlung in eine proximale Endfläche (14) eines Lichtleiters eingekoppelt werden, die aus einer distalen Endfläche (25) des Lichtleiters austretende erste und zweite Beleuchtungsstrahlung mit einem Objektiv (17) auf einen zu beobachtenden Objektbereich eingestrahlt werden, eine von dem Objektbereich ausgehende Beobachtungsstrahlung von dem Objektiv (17) aufgenommen und in die distale Endfläche (25) des Lichtleiters eingekoppelt wird und die aus der proximalen Endfläche (14) des Lichtleiters austretende Beobachtungsstrahlung einer Detektoreinrichtung (20, 21) zugeführt wird, wobei ein Anteil der Beobachtungsstrahlung im ersten Wellenlängenbereich zur Erfassung von optisch-kohärenztomographischen Bilddaten ausgewertet wird und ein weiterer Anteil der Beobachtungsstrahlung zur Erfassung von konfokalen Bilddaten ausgewertet wird und wobei der Lichtleiter als eine einzelne Lichtleitfaser ausgebildet ist, deren distale Endfläche (25) zum Scannen des Objektbereichs quer zur optischen Achse des Objektivs (17) bewegt wird.

14. Verfahren nach Anspruch 13, **dadurch gekennzeichnet, dass** die distale Endfläche (25) in zwei Richtungen quer zur optischen Achse (29) des Objektivs (17) bewegt wird und dass aus den optisch-kohärenztomographischen Bilddaten 3D-Daten des Objektbereichs erzeugt werden und/oder aus den konfokalen Bilddaten ein zweidimensionales oberflächennahes Bild des Objektbereichs erzeugt wird.
